# EUROPEAN PATENT APPLICATION

(11) **EP 3 640 626 A1**
(43) Date of publication of application: **22.04.2020**
(21) Application number: 18201333.4
(22) Date of filing: 18.10.2018
(51) Int. Cl.: G01N 21/71, G01J 3/443, G01N 33/44

(54) **LASER-INDUCED BREAKDOWN SPECTROSCOPY DEVICE AND USE THEREOF FOR THE ANALYSIS OF CHEMICAL ELEMENTS IN RUBBER**

(71) Applicant: Kraiburg Austria GmbH & Co. KG, 5132 Geretsberg (AT); Johannes Kepler Universität Linz, 4040 Linz (AT)
(72) Inventor: Lackner, Johannes, 5132 Geretsberg (AT); Spendelhofer, Wolfgang, 1230 Wien (AT); Pedarnig, Johannes David, 4020 Linz (AT); Trautner, Stefan, 4020 Linz (AT)
(74) Representative: Eder Schieschke & Partner mbB

(57) **Abstract**

The invention relates to a laser-induced breakdown spectroscopic device for the analysis of chemical elements in rubber, in particular for the analysis of the amount of sulphur and/or zinc in rubber, the device comprising: (a) a double pulse laser configured to irradiate laser light in the visible or ultraviolet range for generating a plasma on the surface of a rubber in ambient conditions (e.g. in air), and (b) a spectrometer (e.g. a grating spectrometer) configured to detect light in the infrared range which is emitted from plasma-excited atoms of the rubber, the spectrometer comprising a detector (e.g. a CCD chip) being capable of time-resolved detection of light in the infrared range. The invention further relates to a method for the evaluation of measured spectra and the quantitative analysis of a chemical element in rubber by using the said laser-induced breakdown spectroscopic device. The device and the method of said invention are applicable to "in-line" measurements in the industrial production of rubber.

## Description

The present invention relates to a laser-induced breakdown spectroscopic device for the analysis of chemical elements in rubber, in particular for the analysis of the amount of sulphur and /or zinc in rubber, which allows the determination of the amount of chemical elements during the in-line production of rubber.

Rubber is produced from various polymers, fillers, oil and various additives of different functionality. In the production of rubber, e.g. rubber for tires, technical rubber goods and conveyors, sulphur is often used as crosslinking agent. For reasons of activation, zinc oxide is used additionally. The amount of added sulphur and zinc oxide (mass fractions are in the range of few wt.%) are to be determined accurately to ensure that the material properties of the rubber are within the desired specifications. Industrial rubber is usually produced in that the raw materials are mixed in an internal mixer, and the resulting mixture is then processed into a continuous rubber sheet, for instance by means of a rolling mill or an extrusion system. For the quantitative determination of elements in the rubber, such as sulphur and zinc, usually a part of the sheet is punched out for subsequent elemental analysis in a laboratory. This procedure has the drawback that the elemental analysis is much slower than the production of the rubber, leading to the problem that the quality of the rubber may not be controlled sufficiently before the rubber is further processed. This may lead to the situation that some or batch or other of the rubber is to be discarded. Since the laboratory analysis is slow, however, the batch may already be further processed. The resulting product is then to be discarded, too. That is, there is a need for a faster elemental analysis of rubber, ideally fast enough for determination during the in-line production of the rubber.

In principle, there are different measurement techniques available for fast determination of elements: X-ray fluorescence (XRF) and Prompt gamma neutron activation analysis (PGNAA) are measurement techniques that are capable of in-line measurements in industrial production. For qualitative analysis, portable XRF devices are developed by different companies (used e.g. for identification of metal scrap). The sensitivity of such hand-held devices for the detection of S and Zn in rubber is not known. X-rays are absorbed in the air and the sample being measured is usually brought in mechanical contact to the device therefore due to the short penetration depth of x-rays. However, mechanical contact may contaminate the sample surface. For quantitative analysis, XRF laboratory devices are used and special sample preparation techniques are usually required. PGNAA is used for quantitative in-line analysis (e.g. of coal). As this measurement method is using radioactive substances and radiation, issues related to radiation protection and workplace safety are to be dealt with.

Another technique for fast elemental analysis is the so-called Laser-induced breakdown spectroscopy (LIBS). However, for the application of this method to the analysis of rubber, it is usually required that the sample to be measured is in vacuum or inert gas atmosphere in order to eliminate interfering signals deriving from oxygen in the air or reaction products of the sample with air, such as C-N compounds, and to avoid the optical absorption in air. Especially when the amount of sulphur is to be determined, the sulphur emission lines in the vacuum ultraviolet spectral range are absorbed in air and the sulphur emission lines in the near infrared spectrum lie in a similar range as the oxygen emissions and the C-N emissions. That is, with conventional LIBS devices the quantitative determination of sulphur in rubber is hardly possible under ambient conditions. However, a measurement method under ambient conditions is necessary for fast quantitative determination of elements during rubber production.

It is therefore the object of the present invention to provide a measurement device and a measurement method which allows the fast quantitative determination of elements in rubber during the rubber production, i.e. under ambient conditions, e.g. in air and without the need for vacuum or inert gas atmosphere, and directly "in-line" of the industrial rubber production. In particular, the quantitative determination of sulphur and zinc in rubber is desired during the in-line production of rubber.

This object is achieved by the provision of a special laser-induced breakdown spectroscopic device (LIBS device) for the analysis of chemical elements in rubber. The device comprises a double pulse laser configured to irradiate laser light in the visible or ultraviolet range for generating a plasma on the surface of the rubber, and a spectrometer configured to detect light in the infrared range which is emitted from plasma-excited atoms of the rubber, the spectrometer (referred to as "IR spectrometer" in the following) comprising a detector being capable of time-resolved detection of light in the infrared range, e.g. a time-gated CCD detector.

The inventors of the present invention found out that the combination of a double pulse laser and a detector having time-resolved detection capacity allows the quantitative determination of sulphur in rubber even if the measurement is carried out under ambient conditions, such as under air, and even if the rubber is moved with a velocity of e.g. 15 cm/s with respect to the LIBS device. The double pulse laser allows the reduction of interfering signals from oxygen and C-N compounds - which are usually present if measurements are conducted in air - for the following reasons: The first pulse of the double pulse produces a plasma on the surface of the rubber, thereby creating a bubble-like area with low pressure. The second pulse of the double pulse then strikes the rubber in the bubble with low pressure and re-excites the plasma. The re-excited plasma is then expanding in the area of low pressure, i.e. in an area where the pressure of air is lower compared to an area of ambient pressure. That is, the interfering influence of oxygen is thereby reduced. The detector having time-resolved detection capacity allows detecting signals of emitted wavelengths in a special time window. This is necessary to prevent that the molecular emissions of the plasma overlie the atomic emissions of the element, e.g. of sulphur.

In an embodiment of the present invention, the LIBS device comprises an optical lens (referred to as "laser lens" in the following) for focusing the laser light to a focal point (referred to as "laser light focal point" in the following) and a mirror for reflecting the laser light in a certain direction being defined as X-axis (referred to as "laser mirror" in the following), wherein the laser lens is closer to the laser than the laser mirror with respect to the optical path of the laser light. The laser lens is necessary to bundle the energy of the laser light to one point, namely, the laser light focal point. The LIBS device may further have optical components for expanding the laser light before being bundled and focused by the laser lens. The laser mirror provides the advantage that the laser may be arranged in a position different from the opposite side of the laser light focal point. The latter may be advantageous if the laser and further components of the LIBS device are to be arranged together in a compact housing, especially if the compact housing should only have one opening for passing through the laser light as well as for passing through the light emitted from the sample probe (rubber).

In an embodiment, the LIBS device according to the present invention comprises a further mirror (referred to as "IR mirror" in the following) for reflecting light in the infrared range (IR light) which is emitted in a direction of the X-axis, and a further optical lens (referred to as "IR lens" in the following) for focusing the reflected IR light to a focal point for being detected by the IR spectrometer (referred to as "IR light focal point" in the following). In other words, the IR mirror is arranged in such a way that it reflects light from the sample probe (laser light focal point) in the opposite direction of the laser light. This arrangement has the advantage that if laser and IR spectrometer are located in the same housing, the housing may have only one opening for passing through the laser light as well as for passing through the light emitted from the sample probe. By the before-mentioned arrangement, laser mirror and IR mirror are positioned along the afore-mentioned X-axis. Thereby, the IR mirror may be arranged between laser mirror and laser light focal point. In doing so, it is preferred that the IR mirror is a dichroic mirror which is reflective for IR light and transmissive for the laser light. Alternatively, the laser mirror may be arranged between IR mirror and laser light focal point. In doing so, it is preferred that the laser mirror is a dichroic mirror which is reflective for the laser light and transmissive for IR light.

In an embodiment, the LIBS device according to the present invention comprises a further spectrometer (in the following also referred to as "UV/VIS spectrometer") configured to detect light in the ultraviolet and/or visible range (UV light) which is emitted from plasma-excited atoms of the rubber. Due to the presence of the further spectrometer, spectral signals of chemical elements emitting UV light may be detected and the quantity of such elements in the rubber may be determined. In doing so, the quantity of Zn in the rubber may also be determined together with the quantity of sulphur.

In the latter embodiment, the LIBS device according to the present invention may comprise a further mirror for reflecting UV light (referred to as "UV mirror" in the following) which is emitted in a direction of the X-axis, and a further optical lens for focusing the reflected UV light (referred to as "UV lens" in the following) to a focal point for being detected by the further spectrometer (referred to as "UV light focal point" in the following).

In an embodiment of the LIBS device according to the present invention, optical lenses, either laser lens and IR lens, or laser lens, IR lens and UV lens, are arranged such that their optical axes are essentially parallel to each other. The term "essentially" shall comprise cases in which the parallelism deviates up to a 5° angle between the optical axes of two lenses. This again has the advantage of a possible compact design if the major components of the LIBS device are integrated in a housing.

It is further preferred that laser mirror, IR mirror and UV mirror are positioned along the afore-mentioned X-axis. Every sequence of the three mirrors is conceivable: It is, however, most preferred that the laser mirror is positioned between the IR mirror and the UV mirror. If the UV mirror is located behind and the IR mirror is located in front of the laser mirror with respect to the laser light focal point, the laser mirror is a dichroic mirror reflecting the laser light and transmitting the UV light. In this case, the IR mirror is also a dichroic mirror reflecting IR light and transmitting laser light and UV light. If the IR mirror is located behind and the UV mirror is located in front of the laser mirror with respect to the laser light focal point, the laser mirror is a dichroic mirror reflecting the laser light and transmitting the IR light. In this case, the UV mirror is also a dichroic mirror reflecting UV light and transmitting laser light and IR light.

Furthermore, the LIBS device according to the present invention may comprise one or more optical fibers for transportation of detected light to the IR spectrometer or the further spectrometer. One end of the optical fiber is then preferably located at the IR light focal point or the UV light focal point and the other end of the optical fiber ends in the corresponding spectrometer. If the LIBS device according to the invention comprises two spectrometers, it is preferred that each spectrometer is coupled with an optical fiber for the transportation of detected light to each spectrometer. One optical fiber transports the IR light from the IR light focal point to the IR spectrometer and the other optical fiber transports the UV light from the UV light focal point to the further spectrometer. The presence of optical fibers further allows a compact design of a housing in which all major components of the LIBS device are integrated. Optical fibers usable in the present invention are multimode optical fibers, e.g. made of quartz glass. In an embodiment, the LIBS device according to the present invention comprises a housing in which at least the laser and the IR spectrometer are integrated. Preferably the housing also comprises the laser lens, the laser mirror, the IR lens and the IR mirror. If present, the housing also comprises the optical fiber which is connected to the IR spectrometer. If the LIBS device according to the invention also comprises the further spectrometer, it is preferred that the further spectrometer is also integrated in said housing. It is then preferred that the UV lens and UV mirror are also integrated in said housing. If present, the housing also comprises the optical fiber which is connected to the further spectrometer. It is particularly preferred that all optical components, such as laser spectrometer, lenses, mirrors, optical fibers are located in the housing. Power supply units of the electronic components, such as laser and spectrometer, may not be integrated in the housing.

The housing is preferably hermetically sealed and comprises an optical aperture for passing light in the direction of the X-axis. It is preferred that the housing only comprises one optical aperture. The optical aperture allows the transmission of laser light as well as the transmission of emitted IR light and - if measured - UV light. The optical aperture is preferably made of a transparent material, such as glass, quartz glass, sapphire or polymer. The reason for the hermetic sealing of the housing is to protect the optical components from getting soiled.

The LIBS device according to the present invention may further contain a means for providing a gas flow between the outer side of the optical aperture of the housing and the laser focal point. This means has the advantage that due to the gas flow, the optical path of the laser beam between optical aperture and laser focal point, and the aperture itself, may be kept free of dust, deposition, etc. In doing so, absorption of laser light and of light emitted from the sample (rubber) is avoided. Without such a gas flow, measurements are possible, however, only for a relatively short period of time due to the fast contamination of the optical aperture. Since the LIBS device according to the present invention may be used for measurements in air, the gas used for the gas flow is preferably air. Using gases other than air is possible, but is not advisable from the viewpoint of costs and handling effort.

The LIBS device according to the present invention may further comprise a data processing equipment which is able to process the data obtained from the IR spectrometer, and - if present - the further spectrometer. It is preferred that the data processing equipment is not located in the housing. The data processing equipment is configured to process the data obtained and to calculate the weight percentage of the desired elements in the sample, preferably rubber, to be measured.

The double pulse laser used in the LIBS device according to the invention is preferably a laser configured to emit light of a wavelength in the range of 100 nm to 800 nm, more preferably in the range of 200 nm to 600 nm. The double pulse laser may be any laser emitting light in the afore-mentioned range. For reasons of space and manageability, it is, however, preferred that the laser is a solid state laser, more preferably a Nd:YAG laser which is frequency-doubled, i.e. emitting light of the wavelength of 532 nm. Using lasers having a wavelength higher than the above range deteriorates the signals of the intensity spectra obtained and results in a less precise determination at least of the amount of sulphur in rubber.

The IR spectrometer is preferably a spectrometer which is at least able to detect IR light, wherein the further spectrometer is preferably one which is at least able to detect UV and/or visible (VIS) light.

The present invention is further directed to a method for the analysis of a chemical element in rubber by using the LIBS device according to the invention. The method according to the invention comprises:
- irradiating double pulses of laser light to the surface of a rubber, thereby generating a plasma on the surface of the rubber; and
- detecting light in the infrared range which is emitted from plasma-excited atoms of the rubber by means of the IR spectrometer;
- determining the quantity of a chemical element in the rubber.

In an embodiment of the method of the present invention, it is preferred that the double pulsed laser has a repetition frequency in the range of 5 double pulses per second to 25 double pulses per second, preferably in the range of 10 double pulses per second to 20 double pulses per second, and most preferred 13 double pulses per second to 17 double pulses per second. A repetition frequency outside the range mentioned deteriorates the signals of the intensity spectra obtained at least with respect to the determination of sulphur. For the same reasons, it is preferred that the pulse duration of each of the double pulses is in the range of 1 ns to 20 ns, and more preferably in the range of 3 ns to 10 ns. The interval between the two pulses of the double pulse is preferably in the range of 0.1 µs to 5 µs, more preferred in the range of 0.5 µs to 2.0 µs, and most preferred in the range of 0.8 µs to 1.2 µs.

For the reason of a suitable detection of elemental signals in a wavelength range in which interfering signals are to be expected, the detector of the spectrometer is detecting light in a time window in the range of 5 µs to 20 µs, the detection starting 2 µs to 10 µs after the first pulse of each double pulse. If time window of detection and pulse frequency and pulse duration are not matched, it is hardly possible to obtain meaningful results with respect to the amount of sulphur in rubber. A possible spectrometer for the present invention is a Czerny Turner grating spectrometer. A possible detector for the present invention is a fast CCD detector with high time resolution.

It is preferred in the method of the present invention that the chemical element to be analyzed is sulphur. Nevertheless, it is also possible to analyze the amount of other chemical elements in rubber with the method according to the invention, including zinc, carbon, silicon, magnesium, manganese, and aluminum. If necessary for the detection of signals in the visible or ultraviolet range, the method of the present invention further comprises the step of detecting light in the ultraviolet or visible range via the further spectrometer, especially for the quantitative determination of Zn in the rubber. The further spectrometer may be any standard UV/VIS spectrometer known in the respective field, such as e.g. an Echelle spectrometer.

It is particularly preferred in the method of the present invention that the amount of sulphur and the amount of zinc is determined. The amount of sulphur is determined via the emitted IR light detected by the IR spectrometer, whereas the amount of zinc is determined via the emitted UV light detected by the further spectrometer.

In the method according to the present invention, determining the quantitative amount or concentration of a chemical element in the rubber preferably comprises measuring the intensity of the atomic emission line of the chemical element. Then, the integral Iₑₗₑₘₑₙₜ of the intensity in a spectral window in the range of the atomic emission line of the chemical element is determined. Furthermore, the integral I_{background} of the intensity in a spectral window without the atomic emission line is determined. Then, the standardized signal Iₑₗₑₘₑₙₜ/I_{background} of the chemical element is determined.

The determination of the quantity of the chemical element, especially if the element is sulphur, is in particular possible if calibration curves of rubbers are calculated by measuring rubber samples of varying concentrations of the chemical element. From this information, the data processing equipment can determine the amount of the element in an unknown rubber sample, especially when taking into account the standardized signal Iₑₗₑₘₑₙₜ/I_{background} of the respective element. Moreover, it is preferred that these calibration curves are measured in advance for a certain rubber from which the amount of the element is to be determined.

That is, the method of the present invention comprises the previous determination of a calibration curve for a specific rubber and for the element to be determined, and then calculating the standardized signal Iₑₗₑₘₑₙₜ/I_{background}, and then determining the concentration of the element using the calibration curve. When the method for the analysis of the chemical element is subsequently used for the same specific rubber (having unknown concentrations of elements such as sulphur and zinc), it is possible to determine the amount of the element on the basis of the previously determined calibration curves for the specific rubber. That is, the method of the present invention is particularly suitable for the analysis of rubber for which calibration curves have already been measured and determined. In doing so, it is possible to analyze the amount of a respective chemical element, in particular sulphur, in rubber during the in-line production of the rubber.

The method according to the invention is particularly suitable for the production of a rubber with a specific composition in which, however, the concentration of the crosslinking system, such as sulphur and zinc oxide, may vary due to fluctuations during the in-line production. Since the presence of the amount of sulphur and zinc oxide strongly influences the cross-linking degree and therefore the properties of the resulting rubber, it is very important to control the amount of these ingredients during the production process. This is possible with the method of the present invention.

The rubber used in the present invention is any kind of sulphur cross-linked rubber. The rubber may be natural or synthetic rubber, or a mixture thereof. The rubbers may contain additives which are known to a person skilled in the field of rubbers, depending on the intended use of the rubber. The rubber may be one which is used in the field of tires and tire retreading, which is used for technical rubber goods, and which is used in special applications. For example, tires may be made of mixtures comprising natural and synthetic rubbers. The rubber mixtures may contain synthetic rubbers, such as styrene butadiene rubbers, plasticizers, such as oils, fillers, for example carbon black, and further additives, such as fatigue inhibitors, ozone protection agents, anti-oxidation agents, anti-aging agents and stabilizers. The mixtures may further contain a vulcanizing system for crosslinking the elastomeric components. The vulcanizing system preferably contains sulphur, and zinc oxide as activator. The vulcanizing system may also contain a further activator, such as stearic acid, a vulcanization accelerator and/or retarder.

Depending on the field of application and kind of rubber, rubbers may be produced by a single-stage, double-stage or multiple-stage mixing step of the components to be used for its production. The mixing step may be made in any kind of known mixing devices, such as an internal mixer, and is usually a discontinuous process. After the mixing step, the resulting compound may be further processed to a sheet-like shape. This may be made by a discharging extruder or a roller mill. In doing so, the discontinuous process of mixing is turned into a continuous process.

Only for reasons of exemplifying the rubber production and not for limiting the present invention, a specific production method is described in the following procedure (A): In a first step, the elastomeric components, the filler (preferably carbon black), plasticizer, activators and additives are mixed, preferably in an internal mixer. The mixing time is usually in the range of 3 to 5 minutes, until a temperature of 150°C is reached. The mixture obtained in this way may be stored for several hours or days, and is then further mixed with the sulphur and - if present - the vulcanization accelerator and/or retarder. The latter mixing is also preferably made in an internal mixer, preferably at 80°C to 120°C, a temperature which should not be exceeded for avoiding the cross-linking reaction during the mixing step. After mixing, the resulting compound is rolled in a roller mill, thereby turning the discontinuous process into to a continuous process. The method of the present invention is usually carried out directly after the rubber compound has passed the roller mill.

All information given in this application with respect to the device according to the present invention shall also apply to the method according to the present invention, and vice versa.

The present invention is further illustrated by the following Figures and Examples, but shall not be limited by the Figures and Examples.
- Fig. 1:: Fig. 1 shows a schematic view of a LIBS device according to the present invention.
- Fig. 2:: Fig. 2 shows a LIBS device according to the invention in the form of a measurement head comprising all optical components in a housing.
- Fig. 3:: Fig. 3 is a diagram showing a LIBS spectrum with the measured intensity of Zn emission lines at three different concentrations of ZnO in rubber as a function of the wavelength.

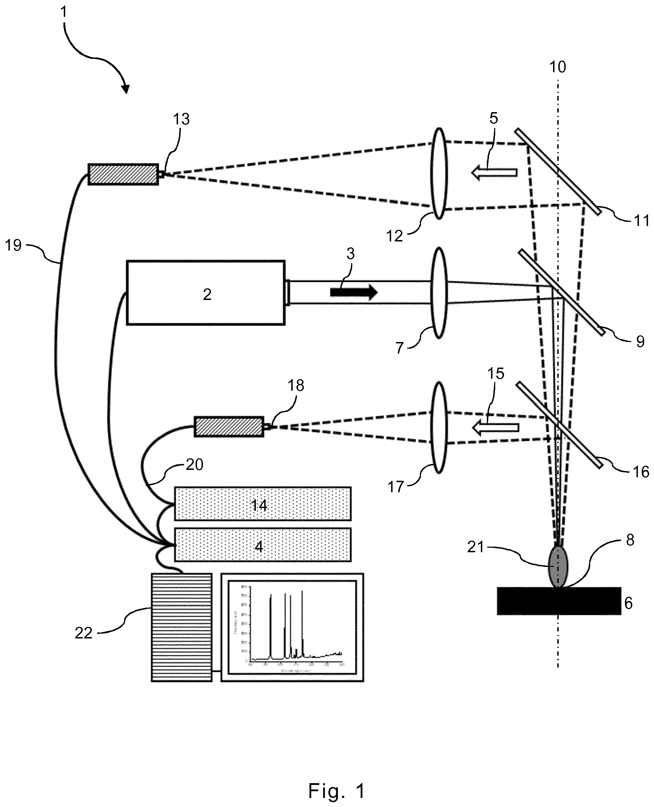

Fig. 1 shows a LIBS device 1 according to the invention comprising a laser 2 as double pulse laser which emits a laser light 3 to the laser lens 7 which focuses the laser light 3 via the laser mirror 9 to a laser light focal point 8 where a rubber 6 is located. The laser light 3 is focused to the focal point 8 along the direction of the X-axis 10. Thereby, plasma 21 of the rubber 6 is produced by the focused laser light 3. Ablated atoms of the rubber 6 are thereby brought into excited states. Due to the relaxation of the atoms from the excited level into lower energetic levels, specific light is emitted. The light emitted from the plasma 21 of the rubber 6 in the direction of the X-axis 10 is either focused to the IR light focal point 13 or the UV light focal point 18. It is preferred that emitted IR light 5 is focused to the IR light focal point 13, and emitted UV light 15 is focused to the UV light focal point 18. The IR light 5 is preferably reflected from the IR mirror 11 to the IR lens 12. The UV light 15 is preferably reflected from the UV mirror 16 to the IR lens 17. The lenses 12 and 17 focus the light to the respective focal points 13 and 18. As shown in Fig. 1, it is preferred that all mirrors 16, 9 and 11 are located essentially parallel to each other along the X-axis 10 in order to ensure that excitation and emission may be made via the same optical axis; this saves space of the LIBS device, especially if all optical components are to be integrated into the same housing. In the same way, the optical axes of the lenses 12, 7 and 17 are essentially parallel to each other for the same reason. The IR light 5 is detected at the focal point 13. The IR light 5 is detected by an IR spectrometer 4, and the IR light 5 is preferably transported from the focal point 13 to the spectrometer 4 via an optical fiber 19. In the same way, the UV light 15 is detected by the further spectrometer 14, and is preferably transported from the focal point 18 to the spectrometer 14 via an optical fiber 20. As shown in Fig. 1, the spectral information obtained by the spectrometers 4 and 14 are evaluated by the data processing equipment 22 in that the spectrometers 4 and 14 are connected with the data processing equipment. Furthermore, the laser 2 is preferably also connected with the data processing equipment 22 in order to be controlled by the same.

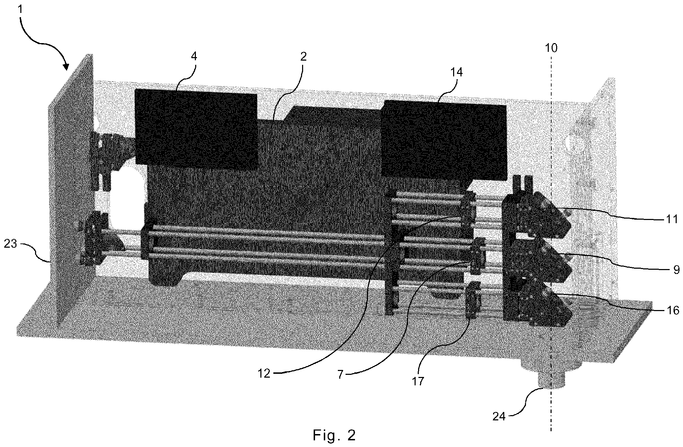

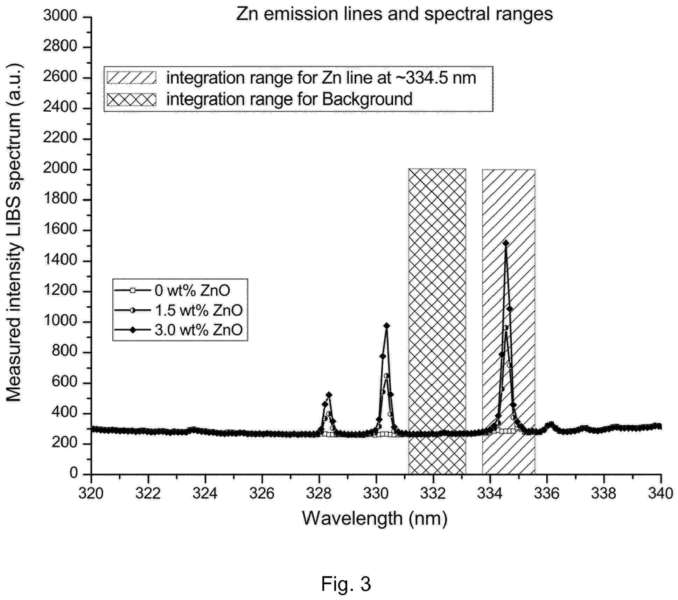

Fig. 2 shows the optical components of a LIBS device 1 according to the present invention integrated in a housing 23. The LIBS device 1 has a laser 2, the light 3 of which may be focused to a focal point along the X-axis 10 by the laser lens 7 via the laser mirror 9 through the optical aperture 24 of the housing 23. Closer to the optical aperture 24 than the laser mirror 9 is preferably located a UV mirror 16 which may reflect emitted UV light to the UV lens 17. In the same way, emitted IR light may be reflected from the IR mirror 11 to the IR lens 12. Via optical fibers (not shown in Fig. 2), the UV and IR light emitted may be transported to the spectrometers 4 and 14 for detection of the emission signals. A LIBS device as shown in Fig. 2 is used for the detection of the LIBS spectrum shown in Fig. 3.

Fig. 3 shows a LIBS spectrum wherein the intensities of the Zn emission lines are shown at three different concentrations (0 wt.%, 1.5 wt.% and 3.0 wt.%) of ZnO in rubbers as a function of the wavelength. The hatched area shows the integration range which is for the Zn line at -334.5 nm. For calculation of the standardized signal Iₑₗₑₘₑₙₜ/I_{background}, the background signal is integrated in the range shown in the checkered area. The three spectra at different ZnO concentrations are measured from rubbers which are identical apart from the amount of ZnO used during production.

The composition for the production of the rubber used for the detection of the LIBS spectrum in Fig. 3 is shown in Table 1:

**Table 1:**

| **Ingredient** | **phr (parts per hundred parts rubber)** | **Supplier (Trade Name)** |
|---|---|---|
| Natural Rubber | 70 | Weber & Schaer (TSR 20) |
| Styrene butadiene rubber | 30 | Arlanxeo (Buna 1500) |
| Carbon black | 60 | Orion Engineered Carbons (Corax N375) |
| Plasticizer | 10 | Shell Oil (Flavex 595) |
| Zinc oxide | variable | Wiehart (Zinkweiss Rotsiegel) |
| Stearic acid | 2 | Nordmann Rassmann (Stearinsäure TP 8) |
| Anti oxidation agent | 1,5 | Lanxess (VULKANOX 4020 LG) |
| Stabilizer | 1 | Lanxess (VULKANOX HS LG) |
| Ozone protection agent | 0,4 | Lanxess (ANTILUX 654) |
| Sulphur | 1,2 | Solvay (Mahlschwefel 80-90) |
| Vulcanization accelerator | 0,1 | Lanxess (RHENOGRAN MBTS-70) |
| Vulcanization accelerator | 1,3 | Lanxess (RHENOGRAN TBBS 80) |
| Retarder | 0,25 | Lanxess (VULKALENT BC) |

The rubber used for the measurement of the spectrum in Fig. 3 is made from the components shown in Table 1 by using the afore-mentioned procedure (A). The amount of zinc oxide is varied (0 wt.%, 1.5 wt.% and 3.0 wt.%). The spectra measured in Fig. 3 at different concentrations of Zn are used to calculate the calibration curve for ZnO in a specific rubber. If afterwards a measurement of the same rubber is made only having a different concentration of Zn (or sulphur), the calibration curves are used for the correct determination of the amount of Zn (or sulphur).

### List of reference signs:

- 1: Laser-induced breakdown spectroscopic device
- 2: Laser
- 3: Laser light
- 4: IR spectrometer
- 5: IR light
- 6: Rubber
- 7: Laser lens
- 8: Focal point (laser light focal point)
- 9: Laser mirror
- 10: X-axis
- 11: IR mirror
- 12: IR lens
- 13: Focal point (IR light focal point)
- 14: Further spectrometer
- 15: UV light
- 16: UV mirror
- 17: UV lens
- 18: Focal point (UV light focal point)
- 19: Optical fiber (IR light optical fiber)
- 20: Optical fiber (UV light optical fiber)
- 21: Plasma
- 22: Data processing equipment
- 23: Housing
- 24: Optical aperture

## Claims

1. A laser-induced breakdown spectroscopic device (1) for the analysis of chemical elements in rubber, the device comprising:
a double pulse laser (2) configured to irradiate laser light (3) in the visible or
ultraviolet range for generating a plasma on the surface of a rubber;
a spectrometer (4) configured to detect light in the infrared range (5) which is emitted from plasma-excited atoms of the rubber (6), the spectrometer (4) comprising a detector being capable of time-resolved detection of light in the infrared range (5).

2. The device (1) of claim 1 comprising an optical lens (7) for focusing the laser light (3) to a focal point (8), and a mirror (9) for reflecting the laser light (3) in a certain direction being defined as X-axis (10), wherein the optical lens (7) is closer to the laser (2) than the mirror (9) with respect to the optical path of the laser light (3).

3. The device (1) of claim 1 or 2 comprising a further mirror (11) for reflecting the light in the infrared range (5) which is emitted in a direction of the X-axis (10), and a further optical lens (12) for focusing the reflected light in the infrared range (5) to a focal point (13) for being detected by the spectrometer (4).

4. The device (1) of any one of claims 1 to 3 which comprises a further spectrometer (14) configured to detect light in the ultraviolet range (15) which is emitted from plasma-excited atoms of the rubber (6).

5. The device (1) of claim 4 comprising a further mirror (16) for reflecting light in the ultraviolet range (15) which is emitted in a direction of the X-axis (10), and a further optical lens (17) for focusing the reflected light in the ultraviolet range (15) to a focal point (18) for being detected by the further spectrometer (14).

6. The device (1) of any one of claims 3 to 5 wherein the optical lenses (7, 12, 17) are arranged such that their optical axes are essentially parallel to each other.

7. The device (1) of any one of claims 1 to 6, further comprising one or more optical fibers (19, 20) for transportation of detected light to the spectrometer (4) or the further spectrometer (14).

8. The device (1) of any one of claims 1 to 7 having a housing (23) which comprises the laser (2) and the spectrometer (4).

9. The device (1) of claim 8, wherein the housing (23) is hermetically sealed and comprises an optical aperture (24) for passing light in the direction of the X-axis (10).

10. The device of any one of claims 1 to 9, wherein the laser (2) is a solid state laser.

11. A method for the analysis of a chemical element in rubber (6) by using a laser-induced breakdown spectroscopic device (1) according to any one of claims 1 to 10, comprising the following:
- irradiating double pulses of laser light (3) to the surface of a rubber (6), thereby generating a plasma (21) on the surface of the rubber (6); and
- detecting light in the infrared range (5) which is emitted from plasma-excited atoms of the rubber (6) by means of the spectrometer (4);
- determining the quantity of a chemical element in the rubber (6).

12. The method of claim 11, wherein the double-pulsed laser light (3) has a repetition frequency in the range of 5 double pulses/s to 25 double pulses/s.

13. The method of claim 11 or 12, wherein the pulse duration of each of the double pulses is in the range of 1 ns to 10 ns.

14. The method of any one of claims 11 to 13, wherein the interval between the two pulses of the double pulse is in the range of 0.1 µs to 5 µs.

15. The method of any one of claims 11 to 14, wherein the detector of the spectrometer (4) is detecting light in a time window in the range of 5 µs to 20 µs, the detection starting 2 µs to 10 µs after the first pulse of each double pulse.

16. The method of any one of claims 11 to 15, wherein the chemical element is sulphur.

17. The method of claim 16, further comprising the step of detecting light in the ultraviolet range (15) via the further spectrometer (14) for the quantitative determination of Zn in the rubber (6).

18. The method of any one of claims 11 to 17, wherein determining the quantitative amount of a chemical element in the rubber (6) comprises measuring the intensity of the atomic emission line of the chemical element, determining the integral Iₑₗₑₘₑₙₜ of the intensity in a spectral window in the range of the atomic emission line of the chemical element, determining the integral I_{background} of the intensity in a spectral window without an atomic emission line, and determining the standardized signal Iₑₗₑₘₑₙₜ/I_{background} of the chemical element.

19. The method of claim 18, wherein the determination of the quantity of the chemical element in rubber (6) comprises the comparison of the measurement results with calibration curves calculated from measurements of rubbers with varying concentrations of the chemical element.

20. The method of any one of claims 11 to 18, wherein the chemical element in rubber (6) is analyzed during the in-line production of the rubber (6).
